# EUROPÄISCHE PATENTSCHRIFT

(11) **EP 2 923 721 B1**
(45) Veröffentlichungstag und Bekanntmachung des Hinweises auf die Patenterteilung: **08.05.2019**
(21) Anmeldenummer: 15000890.2
(22) Anmeldetag: 26.03.2015
(51) Int. Cl.: A61M 16/00, A61M 16/16, F04D 29/66

(54) **VORRICHTUNG ZUR BEATMUNG**
VENTILATION DEVICE
DISPOSITIF RESPIRATOIRE

(30) Priorität: 28.03.2014 DE 102014004850
(43) Veröffentlichungstag der Anmeldung: 30.09.2015
(73) Patentinhaber: Löwenstein Medical Technology S.A., 2557 Luxembourg (LU)
(72) Erfinder: Sepke, Heiko, 22952 Lütjensee (DE); König, Karl-Heinz, 22850 Norderstedt (DE); Feldhahn, Karl-Andreas, 22587 Hamburg (DE); Göbel, Christof, 22457 Hamburg (DE); Gardein, Joachim, 38430 Icod de los Vinos, Tenerife/ Islas Canarias (ES); Kluin, Christian, 20257 Hamburg (DE)
(74) Vertreter: Marx, Thomas

(56) Entgegenhaltungen:
- EP-A2- 2 703 034
- WO-A1-99/22793
- WO-A1-2013/020167
- WO-A1-2013/133889
- US-A1- 2010 307 498
- US-B1- 7 975 688

## Beschreibung

Die Erfindung betrifft eine Vorrichtung zur Beatmung, die ein mit einer Steuerung verbundenes Gebläse aufweist, wobei sowohl die Steuerung als auch das Gebläse in einem Gehäuse angeordnet sind, und wobei die Steuerung mit mindestens einer Anzeigeeinrichtung sowie mindestens einem Bedienelement verbunden ist.

Derartige Vorrichtungen zur Beatmung werden typischerweise zur Energieversorgung an ein Versorgungsnetz angeschlossen und über einen Beatmungsschlauch mit einer Beatmungsmaske verbunden. Ein Patient legt die Beatmungsmaske während der Durchführung der Beatmung im Gesichtsbereich an. Beispielsweise ist es bekannt, derartige Vorrichtungen zur Beatmung zur Durchführung einer CPAP-Therapie, einer APAP-Therapie oder einer Bilevel Beatmung zu verwenden. Grundsätzlich können von der Steuerung des Gerätes beliebige Beatmungsabläufe vorgegeben werden.

Aus dem Stand der Technik sind nachfolgende Vorrichtungen zur Beatmung bekannt, US2010/0307498, WO2013/133889 und EP2703034.

Aufgabe der vorliegenden Erfindung ist gemäß den Merkmalen in Anspruch 1, eine Vorrichtung der einleitend genannten Art derart zu konstruieren, dass eine verbesserte Nutzungsqualität bereitgestellt wird.

Die erfindungsgemäße Vorrichtung zur Beatmung weist ein mit einer Steuerung verbundenes Gebläse auf, wobei sowohl die Steuerung als auch das Gebläse in einem Gehäuse angeordnet sind, und wobei die Steuerung mit mindestens einer Anzeigeeinrichtung sowie mindestens einem Bedienelement verbunden ist und wobei das Gebläse im Gehäuse von einem viskoelastischen oder elastomeren Tragteil gehaltert ist.

Die Vorrichtung ist auch dadurch gekennzeichnet, dass das Gehäuse aus einer Fronteinheit besteht, die die Anzeigeeinrichtung aufweist, und das Gehäuse zwei weitere Gehäuseteile aufweist welche eine Gebläsebox ausbilden.

Die Vorrichtung ist auch dadurch gekennzeichnet, dass das Tragteil zwischen den Gehäuseteilen gehaltert ist, wobei die beiden Gehäuseteile jeweils die anteiligen Kammerhälften von Hochdruckbereich und Ansaugbereich aufweisen.

Die Vorrichtung ist auch dadurch gekennzeichnet, dass das Tragteil in vertikaler Richtung zwischen den Gehäuseteilen angeordnet ist.

Die Vorrichtung ist auch dadurch gekennzeichnet, dass das Tragteil eine Dichtung ausbildet die gegen Dichtflächen der Gehäuseteile abdichtet und das Tragteil eine zweite Dichtung ausbildet, die gegen Dichtflächen der beiden Gehäuseteile abdichtet, die den Hochdruckbereich vom Ansaugbereich pneumatisch trennt.

Die Vorrichtung ist auch dadurch gekennzeichnet, dass das Tragteil in der Gebläsebox der Luftumlenkung und somit der Schallreduzierung dient und dazu zumindest zwei Öffnungen aufweist.

Die Vorrichtung ist auch dadurch gekennzeichnet, dass das Tragteil zumindest eine Öffnung aufweist, die zumindest abschnittsweise von einem Kragen umgeben ist.

Die Vorrichtung ist auch dadurch gekennzeichnet, dass das Tragteil der Lenkung der Luftströme dient und dazu Öffnungen oder Durchbrüche aufweist und das Tragteil ebenfalls der Schalldämmung dient und dazu zumindest an einer Öffnungen einen Kragen aufweist und das Tragteil ebenfalls der Schallentkopplung und Halterung des Gebläses dient und daher aus einem elastomeren Werkstoff gefertigt ist und dazu ein Kopplungselement zur Halterung des Gebläses aufweist, wobei das Kopplungselement kann zudem Sicken aufweisen kann, und das Tragteil zudem mindestens eine Dichtung zur Abdichtung der Gehäuseteile gegenüber der Umgebung aufweist und eine weitere Dichtung zur Abdichtung von Ansaug- und Hochdruckbereich aufweist und das Tragteil ist zudem durch Verklemmung oder Verspannung oder Verschraubung zwischen den Gehäuseteile gehalten wird.

Die Vorrichtung ist auch dadurch gekennzeichnet, dass innerhalb der Gebläsebox für eine Atemgasströmung mindestens drei Umlenkungen angeordnet sind.

Die Vorrichtung ist auch dadurch gekennzeichnet, dass innerhalb der Gebläsebox und/oder am Tragteil mindestens ein schalldämmendes Element angeordnet ist.

Die Vorrichtung ist auch dadurch gekennzeichnet, dass in der Fronteinheit die Anzeigeeinrichtung sowie eine Frontfolie aufgenommen ist, die über mindestens ein Bedienelement verfügt.

Die Vorrichtung ist auch dadurch gekennzeichnet, dass eine Anzeigeeinrichtung geneigt zur vertikalen Richtung angeordnet ist.

Die Vorrichtung ist auch dadurch gekennzeichnet, dass das Gehäuse eine dreieckige Querschnittfläche aufweist

Die Vorrichtung ist auch dadurch gekennzeichnet, dass die Fronteinheit aus einem sich vertikal erstreckenden ersten Segment und einem zweiten zur vertikalen Richtung geneigten Segment besteht, in dem eine Anzeigeeinrichtung angeordnet ist.

Die Vorrichtung ist auch dadurch gekennzeichnet, dass die Anzeigeeinrichtung als ein plattenartiges Display ausgebildet ist, das von einem rahmenartigen Halterungselement in der Fronteinheit fixiert ist.

Die Vorrichtung ist auch dadurch gekennzeichnet, dass Halterungselement einen Klemmbügel zur Fixierung des Displays vorsieht oder dass das Halterungselement in der Fronteinheit verschraubbar ist.

Die Vorrichtung ist auch dadurch gekennzeichnet, dass lediglich ein mechanisches Bedienelement zur Aktivierung einer Gerätefunktion vorgesehen ist.

Die Vorrichtung ist auch dadurch gekennzeichnet, dass ergänzend zum lediglich einen mechanischen Bedienelement weitere Bedienfelder auf dem als Touchscreen ausgebildeten Display vorgesehen sind.

Die Vorrichtung ist auch dadurch gekennzeichnet, dass innerhalb des Gerätes eine Leitungsführung zum Anschluss einer Druckmessung aus einem Leitungsabschnitt eines ersten Durchmessers und einem Leitungsabschnitt eines zweiten Durchmessers ausgebildet ist, wobei der zweite Durchmesser kleiner als der erste Durchmesser ist.

In den Zeichnungen sind Ausführungsbeispiele der Erfindung schematisch dargestellt. Es zeigen:
- Fig. 1: Eine perspektivische Darstellung eines Gerätes mit schräg angeordnetem Display,
- Fig. 2: eine perspektivische Darstellung einer geänderten Gerätekonfiguration,
- Fig. 3: eine perspektivische Darstellung einer Fronteinheit und einer montierten Gebläsebox,
- Fig. 3a: eine weitere perspektivische Darstellung einer Fronteinheit und einer montierten Gebläsebox,
- Fig. 4: eine perspektivische Darstellung eines innerhalb der Gebläsebox angeordneten Gebläses, wobei das Gebläse von einer umlaufenden Membranstruktur gehaltert ist,
- Fig. 4a: eine weitere perspektivische Darstellung des von einer umlaufenden Membran gehalterten Gebläses,
- Fig. 5: eine perspektivische Darstellung einer teilweise auseinandergenommenen Gebläsebox mit eingezeichneter Luftführung,
- Fig. 6: eine Darstellung entsprechend Fig. 5 ohne eingezeichnete Luftführung,
- Fig. 7: eine perspektivische Innenansicht eines Gehäuseteiles,
- Fig. 8: eine perspektivische Darstellung zur Veranschaulichung einer Halterung einer als Display ausgebildeten Anzeigeeinrichtung innerhalb des Gehäuses unter Verwendung eines Rahmens,
- Fig. 9: eine weitere Darstellung des Displays und des Halterahmens,
- Fig. 10: eine kombinierte Darstellung des Displays und des Halterahmens,
- Fig. 11: eine weitere perspektivische Darstellung des Beatmungsgerätes mit zugeordneter externer Bedieneinheit und
- Fig. 12: eine Draufsicht auf eine innerhalb des Gerätegehäuses angeordnete Steuerungsplatine mit Druckmessschlauch.

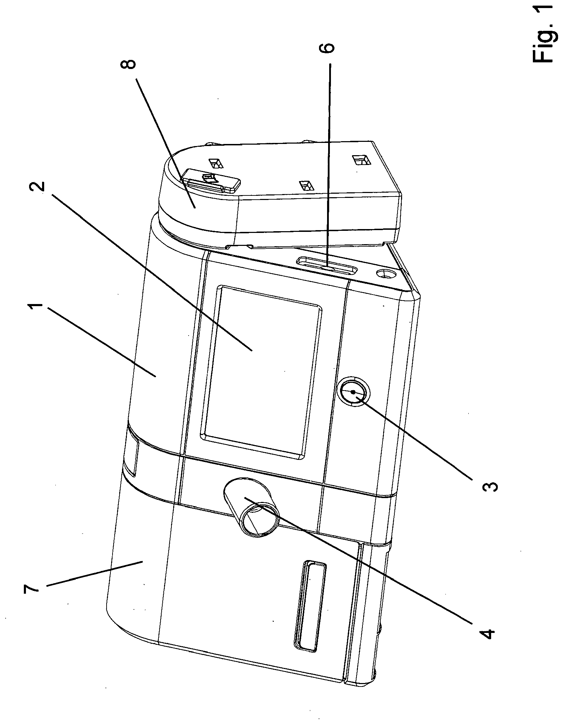

Fig. 1 zeigt ein Beatmungsgerät (1). Das Beatmungsgerät (1) ist mit einer Anzeigeeinrichtung (2), mindestens einem Bedienelement (3) sowie einem Schlauchanschluss (4) versehen.

Der Schlauchanschluss (4) dient typischerweise zur Verbindung mit einem nicht dargestellten Atemgasschlauch, der im Bereich seiner dem Schlauchanschluss (4) abgewandten Ausdehnung mit einer ebenfalls nicht dargestellten Atemmaske verbunden ist. Die nicht dargestellte Atemmaske dient zur Atemgasversorgung eines ebenfalls nicht dargestellten Patienten. Im Bereich des Schlauchanschlusses kann optional ein (nicht dargestellter) elektrischer Anschluss zur Beheizung eines Atemschlauches angebracht sein.

Das Beatmungsgerät (1) ist typischerweise mit mindestens einer Kommunikationsschnittstelle (6) versehen. Die Schnittstelle (6) ist hier in einem Seitenbereich angeordnet. Die Schnittstelle (6) ist zur Horizontalen geneigt und befindet sich in räumlicher Nähe zum Display. Bevorzugt weist das Beatmungsgerät zudem eine Schnittstelle (6') auf die der Kopplung von Modulen (8) dient.

Bei dem dargestellten Ausführungsbeispiel ist das Beatmungsgerät (1) mit einem Atemgasbefeuchter (7) gekoppelt. Das Beatmungsgerät (1) kann optional sowohl mit als auch ohne den Atemgasbefeuchter (7) betrieben werden.

Gemäß der im Fig. 1 dargestellten Ausführungsform weist das Gehäuse des Beatmungsgerätes (1) in einer senkrechten Schnittebene eine dreieckartige Querschnittfläche auf.

Gemäß der Ausführungsform in Fig. 1 können an das Beatmungsgerät (1) bedarfsabhängig Zusatzelemente (8) angekoppelt werden, die mindestens eine weitere Gerätefunktion bereitstellen. Das Display (13) ist hier als Benutzerschnittstelle, beispielsweise als Touchscreen, des Beatmungsgerätes (1) ausgeführt. Im Bereich des Displays (13) sind mehrere Eingabetasten vorgesehen, die zum Empfangen einer Benutzereingabe konfiguriert sind. Das Display (13) ist schaltlogisch mit den Tasten verbunden und weist mehrere Informationsfelder auf wobei jedes Informationsfeld einer entsprechenden Eingabetaste zugeordnet ist und dazu konfiguriert ist, graphische Informationen anzuzeigen, die verschiedene Funktionen der entsprechenden Eingabetasten angeben. Die Eingabetasten sind berührempfindliche Flächen im Bereich des Displays (13) Erfindungsgemäß ist mindestens ein weiteres Bedienelement (3)vorgesehen. Dieses Bedienelement (3) ist mechanisch bedienbar und unterscheidet sich somit von den Berührflächen auf dem Display. Erfindungsgemäß wird das Beatmungsgerät über das Bedienelement (3) ein- und ausgeschaltet, sodass eine grundlegende, patientengerechte Therapie mittels lediglich einer Taste aktiviert werden kann. Dazu ist das Bedienelement (3) schaltlogisch mit dem Gebläsemotor verbunden und aktiviert diesen, sowie den Speicher um hinterlegte Therapiedaten, wie Druckwerte, abzurufen und anzuwenden. Ergänzend kann über die Eingabetasten eine Anpassung einzelner Werte, wie Druckwerte, erfolgen. Eine Bedienung über die Eingabetasten des Touchscreens (13) ist erfindungsgemäß jedoch nicht notwendig, um die Therapie zu starten.

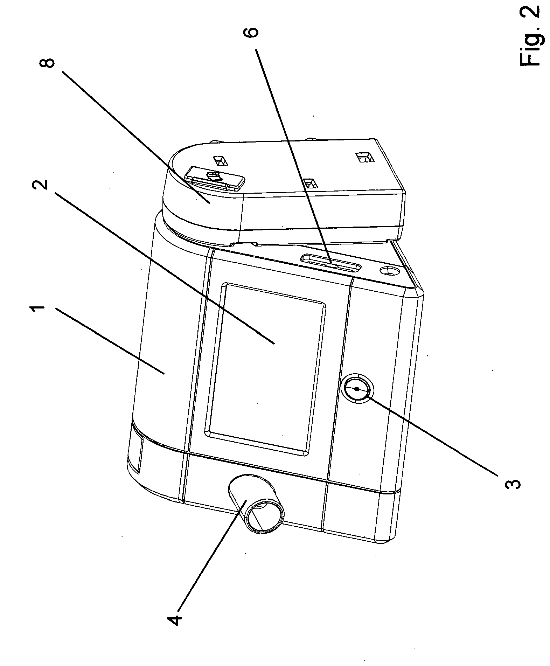

Gemäß der in Fig. 2 dargestellten Ausführungsform ist, bei der Geräteanordnung gemäß Fig. 1, der Atemgasbefeuchter (7) vom Beatmungsgerät (1) abgenommen worden.

Bei einer Zusammenschau von Fig. 1 und Fig. 2 ist erkennbar, dass unabhängig von der Verwendung eines Atemgasbefeuchters (7) immer der gleiche Schlauchanschluss (4) verwendbar ist. Es ist somit nicht erforderlich, abhängig von der Gerätefunktion den Beatmungsschlauch umzustecken.

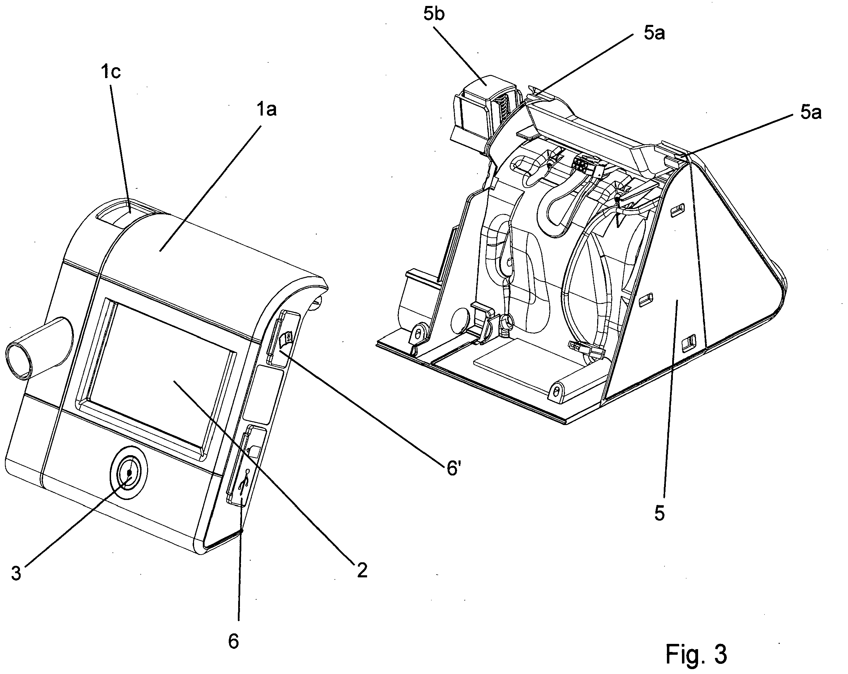

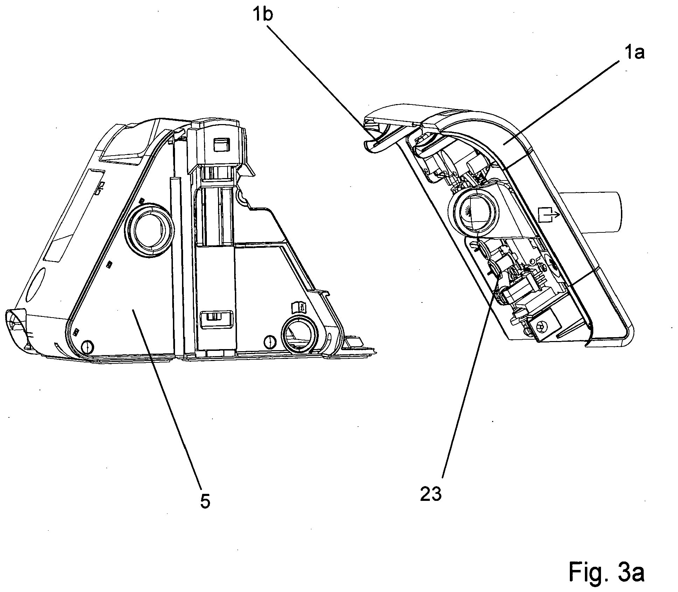

Fig. 3 veranschaulicht, dass das Beatmungsgerät (1) hinsichtlich seiner mechanischen Grundstruktur aus zwei Bauelementen besteht. Dieses sind eine Fronteinheit (1a) mit dem Bedienelement (3) sowie der Anzeigeeinrichtung (2) und der Gebläsebox (5), mit einem Vorderteil (16), einem Tragteil (11) mit dem Gebläse (9) und einem hinteren Teil (17). Das Vorderteil (16) und das Hinterteil (17) umschließen schalenartig einen Geräteinnenraum (18).In die Gehäuseteile (16) und (17) sind schalldämmende Materialien (16', bzw. 17') bevorzugt aus einem Schaumstoff eingesetzt.

Fig. 3 und 3a zeigen das Beatmungsgerät (1) zerlegt in die Bauteile Gebläsebox (5) und Fronteinheit (1a). Aus den Figuren wird die einfache Handhabung bei der Montage der Bauteile ersichtlich. Die Fronteinheit (1a) enthält u.a. neben dem Gehäuse eine Anzeigeeinrichtung (2), ein Bedienelement 3) und eine Steuerplatine (23), sowie zumindest zwei Schnittstellen. Bevorzugt ist die Fronteinheit (1a) zur Vertikalen geneigt und enthält die Anzeigeeinrichtung (2), zumindest ein Bedienelement (3) und zumindest eine Steuerplatine (23), die parallel zur Anzeigeeinrichtung (2) angeordnet ist, sowie zumindest zwei Schnittstellen. Die Montage der Fronteinheit (1a) mit der Gebläsebox (5) erfolgt über Haken (1b) an der Froneinheit (la), die hinter Bereiche (5a) der Gebläsebox (5) greifen. Die Fronteinheit wird über den Drehpunkt aus den Haken (1b) und den Bereichen (5a) eingeschwenkt. Zusätzlich greift ein an der Gebläsebox (5) angeordneter Schnapper (5b) in eine Öffnung (1c) der Fronteinheit (1a). Zur Sicherung wird die Fronteinheit (1a) lediglich mit 2 Schrauben mit der Gebläsebox (5) verbunden und bildet so das Gehäuse des Beatmungsgerätes (1). Zum Lösen der Froneinheit (1a) von der Gebläsebox (5) müssen nur die Schrauben gelöst werden und der Schnapper (5b) entriegelt werden.

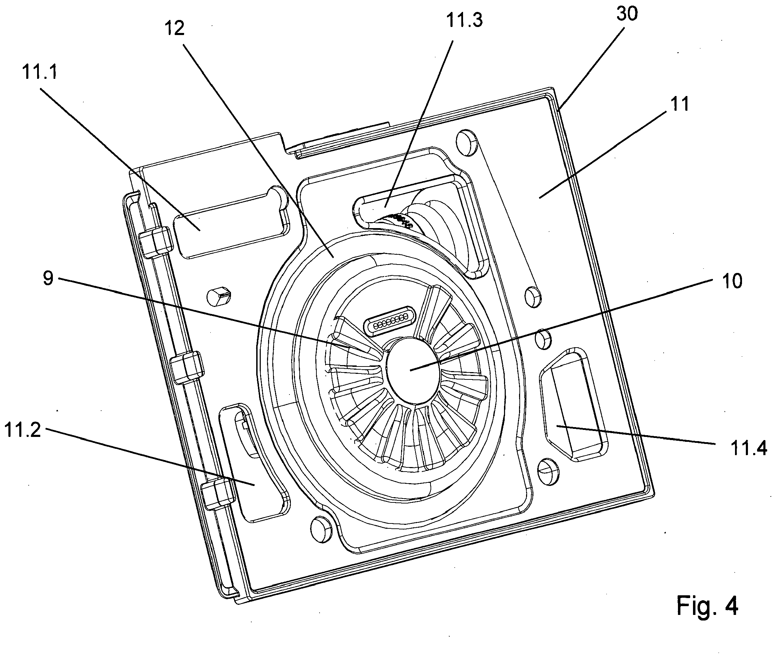

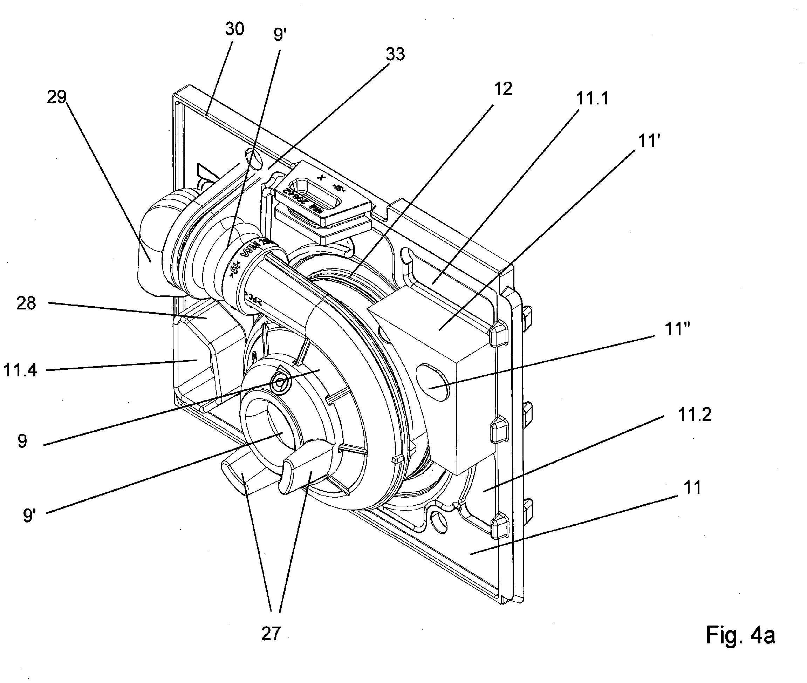

Gemäß der Ausführungsform in Fig. 4 ist innerhalb der Gebläsebox (5) ein Gebläse (9) angeordnet. Das Gebläse (9) wird typischerweise von einem Elektromotor (10) angetrieben.

Zur Unterstützung einer einfachen Montage und Demontage kann das Gebläse (9) von einem - vorzugsweise elastischen oder viskoelastische - Tragteil (11) gehaltert sein, das in die Gebläsebox (5) einsetzbar ist.

Zur Unterstützung einer Körperschallentkopplung ist das Gebläse (9) durch ein ebenfalls (visko)elastisches Kopplungselement (12) mit dem Tragteil (11) verbunden. Bevorzugt sind Tragteil (11) und Kopplungselement (12) einteilig ausgeführt. Diese können beispielsweise aus einem elastomeren Material ausgeführt sein. Elastomere Materialien haben den Vorteil, dass sie sowohl über elastische also auch viskose Eigenschaften verfügen und damit Schwingungsenergie absorbieren bzw. in Wärme umwandeln. Gemäß einer bevorzugten Ausführungsform umgibt das Kopplungselement (12) das Gebläse (9) rahmenartig. In Abhängigkeit von den jeweiligen Anwendungsanforderungen kann das Kopplungselement (12) ringartig gestaltet sein. Vorzugsweise weist das Kopplungselement (12) eine Sicke oder ein ähnliches Gestaltungselement auf - beispielsweise ähnlich zu elastischen Aufhängungselementen, die bei Lautsprechern verwendet werden - um über einen längeren Entkopplungsweg Schwingungen besser absorbieren zu können Das Gebläse (9) durch das (visko)elastische Kopplungselement (12) und/der das Tragteil (11) elastisch im Geräteinnenräum aufgehängt. Dadurch wird eine Körperschallübertragung auf das Gehäuse (1, 16, 17) effektiv vermieden. Zusätzlich können elastische Distanzelemente (27), die bevorzugt einteilig mit dem Kopplungselement (12) und/der dem Tragteil (11) ausgeführt sind, vorgesehen sein, die einen Kontakt des Gebläses mit dem Gehäuse (1, 16, 17) effektiv vermeiden. Die Distanzelemente können dazu einseitig oder beidseitig am Kopplungselement (12) und/der dem Tragteil (11) vorgesehen sein und erstrecken sich beispielsweise in vertikaler Richtung.

Das Tragteil (11) mit dem Kopplungselement (12) besteht bevorzugt aus einem viskoelastischen Material, beispielsweise aus Silikon.

Das Tragteil (11) enthält außer dem Kopplungselement (12) verschiedene Luftöffnungen (11.1, 11.2, 11.3, 11.4) um einen längeren Luftweg und somit eine weitere Schallreduzierung zu erzielen. Auch weitere Umlenkungen der Strömung können mittels des Kopplungselementes erzielt werden.

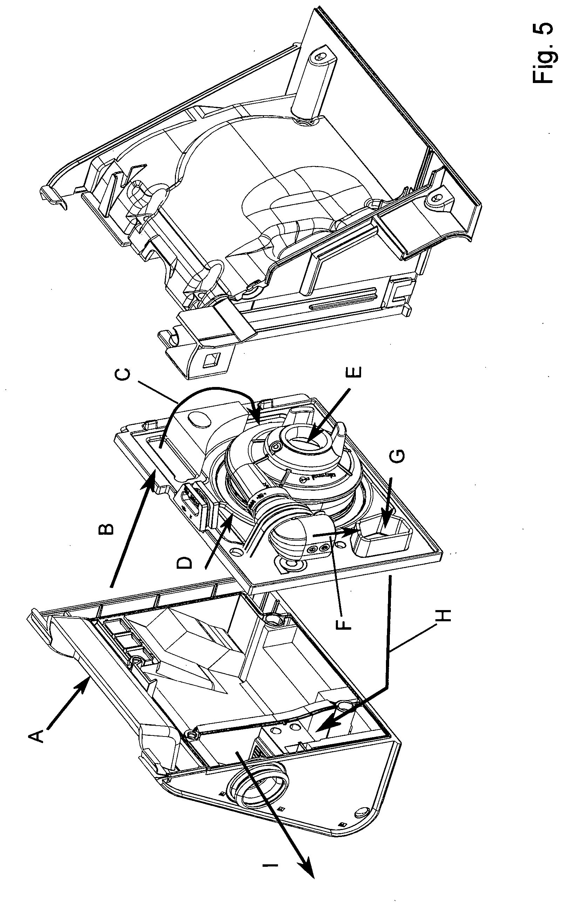

Das Tragteil (11) dient neben der Aufnahme des Gebläses (9) und der gezielten Gasströmung durch den Geräteinnenraum (18) der Gebläsebox (5) welches in Fig. 5 veranschaulicht wird auch der Abdichtung zwischen den Gehäuseteilen (16) und (17). Die Außenkontur des Tragteiles (11) nimmt die Kontur der Gehäuseteile (16 und 17) auf und dichtet so im Randbereich zwischen den Gehäuseteilen (16 und 17) ab. Bevorzugt wird das Tragteil (11) vertikal zwischen den Gehäuseteilen (16 und 17) eingespannt oder eingelegt und durch Fixierung der Gehäuseteilen (16 und 17) gegeneinander (beispielsweise durch Verschraubung) gehalten. Durch die viskoelastischen Eigenschaften des Tragteils (11) werden so die Gehäuseteile (16 und 17) abgedichet. Das Tragteil weist dazu eine beispielsweise umlaufende oder unterbrochene Dichtung (30), beispielsweise als Kante ausgeführt, auf. Die Gehäuseteilen (16 und 17) weisen komplementär zur der Dichtkante (30) Dichtflächen (31, 32) auf.
Das Tragteil (11) dient der Lenkung der Luftströme und weist dazu Öffnungen oder Durchbrüche (11.1, 11.2, 11.3, 11.4) auf. Das Tragteil (11) dient ebenfalls der Schalldämmung und weist dazu zumindest an einer Öffnungen (11.4) einen Kragen (28) auf. Das Tragteil (11) dient ebenfalls der Schallentkopplung und Halterung des Gebläses ist daher aus einem elastomeren Werkstoff gefertigt und weist dazu ein Kopplungselement (12) zur Halterung des Gebläses auf. Das Kopplungselement (12) kann zudem Sicken aufweisen, um die Übertragung von Körperschall zu vermindern. Das Tragteil (11) weist zudem mindestens eine Dichtung (30) zur Abdichtung der Gehäuseteile (16 und 17), über die Dichtflächen (31, 32), gegenüber der Umgebung auf und eine weitere Dichtung (33) zur Abdichtung von Ansaug- und Hochdruckbereich über die Dichtflächen (34, 35) auf. Das Tragteil ist zudem einteilig ausgeführt und wird durch Verklemmung oder Verspannung oder Verschraubung zwischen den Gehäuseteile (16 und 17) gehalten.

Fig. 4a zeigt das Tragteil (11) in einer weiteren Ansicht. Hier wird der Ansaugbereich (9') und der Luftauslass (9") des Gebläses (9) sichtbar. Ein zusätzliches Schaumstoffelement (11'), welches mittels eines Zapfens (11") auf dem Tragteil (11) gehalten wird, dient der Luftumlenkung und somit der Schallreduzierung.

Fig. 5 veranschaulicht eine typische Luftströmung durch die Gebläsebox (5) hindurch. Gemäß A wird Luft durch eine Ansaugöffnung im Hinterteil (16) angesaugt und strömt gemäß B durch die Öffnung (11.1) im Tragteil (11). Gemäß C wird die Luftströmung durch eine Kammer (17.1) im Vorderteil (17) vertikal umgelenkt und tritt durch die Öffnung (11.2) im Tragteil (11) zurück in das Hinterteil (16) in die Luftkammer im Bereich (16.2). Dort wird die Luft diagonal umgelenkt und strömt gemäß D durch die Öffnung (11.3) und diagonal umgelenkt weiter gemäß E im Bereich (9') in das Gebläse (9) ein. Die vom Gebläse (9) geförderte Luft verlässt das Gebläse durch den Luftauslass (9") gemäß F im Bereich (17.2) im Vorderteil (17) weiter gemäß G durch die Öffnung (11.4) im Tragteil (11). Gemäß H erreicht die Luftströmung das Hinterteil (16) im Bereich (16.1) durchströmt dort eine Flow-Messeinrichtung und wird gemäß I erneut umgelenkt und verlässt das Hinterteil (16) zu einem Schalldämpfer oder zum Atemgasbefeuchter.

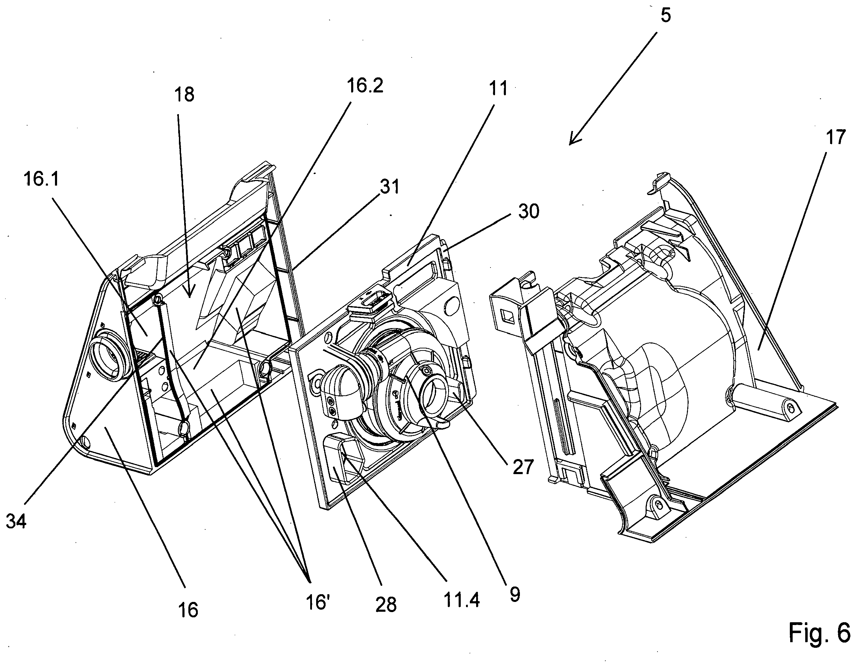

Fig. 6 zeigt die Bauteile gemäß Fig. 5 zur näheren Erläuterung der mechanischen Konstruktion ohne die eingezeichneten Strömungspfeile der Gasströmung. Das Tragteil (11) weist bevorzugt zumindest eine Öffnung oder einen Durchbruch (11.1, 11.2 ...) auf der der Luftlenkung dient. Das Tragteil (11) weist bevorzugt zumindest eine Öffnung oder einen Durchbruch (11.4) auf der zumindest abschnittsweise von einem erhöhten Kragen (28) umgeben ist. Der Kragen kann einseitig oder beidseitig vom Durchbruch (11.4) vorhanden sein. Der Kragen (28) ist einteilig mit dem Tragteil (11) verbunden und dient der Luftlenkung und der Dämmung von Luftschall. Die vom Gebläse (9) geförderte Luft verlässt das Gebläse durch den Luftauslass (9"). Kurz hinter dem Luftauslass wird die beschleunigte Luft durch einen Strömungsumlenker (29) um etwa 90° umgelenkt und so in Richtung auf den Durchbruch (11.4) mit Kragen (28) führt.

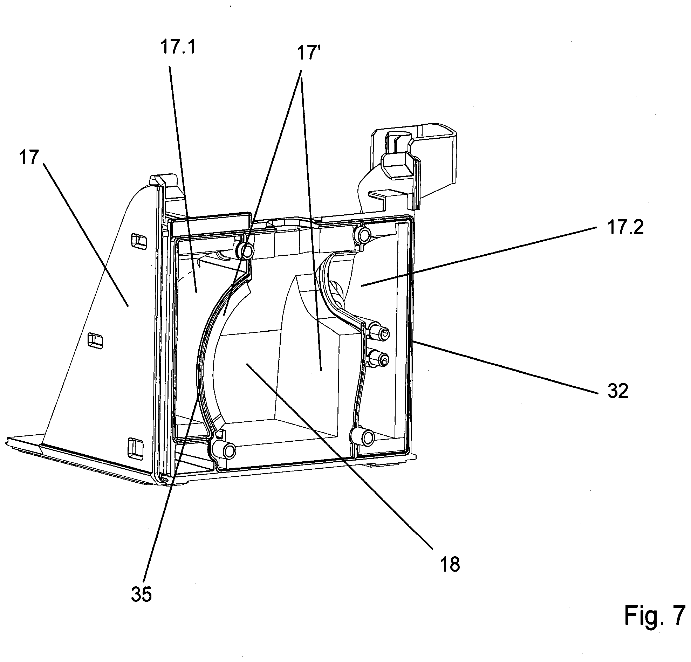

Fig. 7 zeigt den Gehäuseinnenraum (18) in dem Vorderteil (17) mit eingesetzten schalldämmenden Materialien (17') und den Luftkammern (17.1) und (17.2). Erfindungsgemäß sind die Luftkammern (17.1) und (17.2) der Hochdruckbereich (17.1) und der Niederdruckbereich oder Ansaugbereich (17.2). Entsprechend weisen die beiden Gehäuseteile (16, 17) jeweils die anteiligen Kammerhälften von Hochdruckbereich (16.1, 17.1) und Ansaugbereich (16.2, 17.2) auf.

Das Tragteil (11) weist zur Trennung von Hochdruckbereich (16.1, 17.1) und Ansaugbereich (16.2, 17.2) eine Dichtung (33) auf, die vertikal zwischen den Gehäuseteilen (16 und 17) eingespannt und durch Fixierung der Gehäuseteilen (16 und 17) gegeneinander (beispielsweise durch Verschraubung) gehalten wird. Die Gehäuseteilen (16 und 17) weisen komplementär zur der Dichtung (33) Dichtflächen (34, 35) auf.

Im Zusammenspiel zwischen dem Tragteil und den Gehäuseteilen (16, 17) ergeben sich so unterschiedliche gegeneinander abgedichtete Räume. Das Tragteil (11) dichtet den Hochdruckbereich (16.1, 17.1) vom Ansaugbereich (16.2, 17.2) ab. Zudem trennt das Tragteil (11) die beiden Gehäuseteile (16, 17) ab. Somit werden vier Kammern geschaffen, die durch die Öffnungen (11.1, 11.2, 11.3, 11.4) miteinander kommunizieren und die Luft lenken.

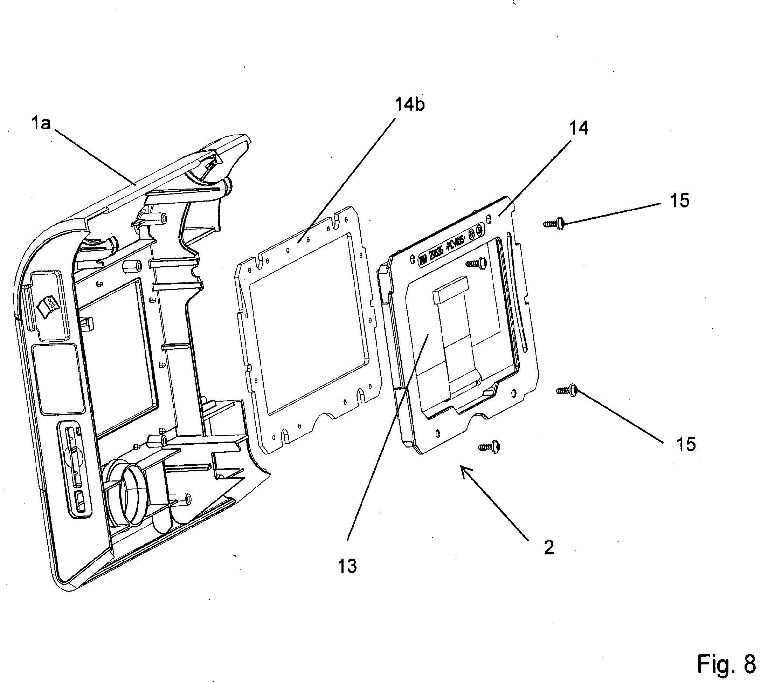

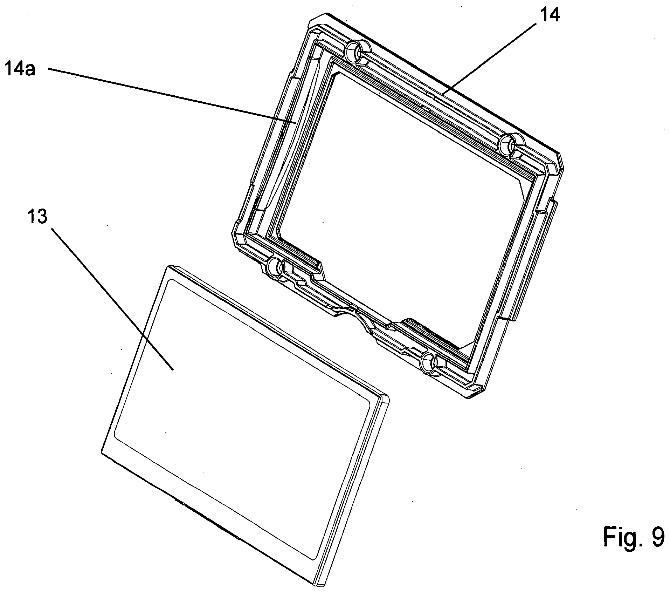

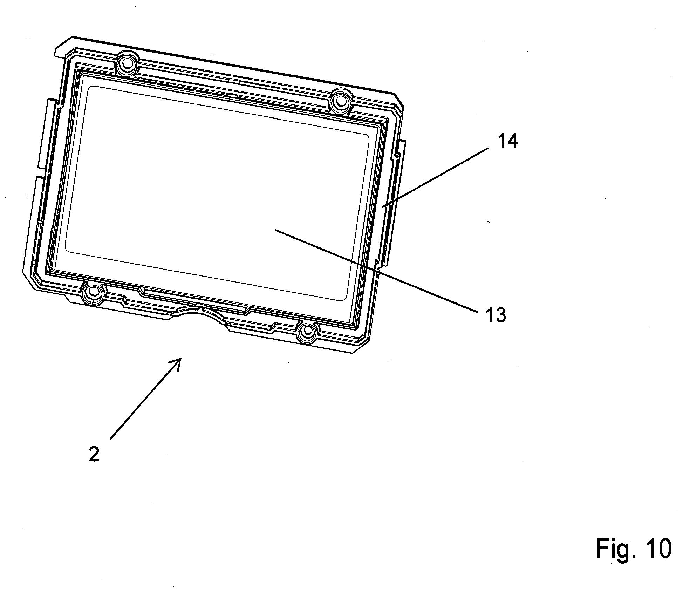

Gemäß der Ausführungsform in Fig. 8 ist die Anzeigeeinrichtung (2) als ein Display (13) ausgebildet. Das Display (13) ist plattenartig gestaltet und wird mittels eines rahmenartigen Halterungselements (14) innerhalb des Gehäuses (5) fixiert. Vorzugsweise ist daran gedacht, dass das Display (13) in das Halterungselement (14) eingesetzt und mittels eines Klemmbügels (14a) in dem Halterungselement (14) fixiert wird. Dieses dient der besseren Fixierung des Displays im Gehäuse, da das Display über Kopf in das Gehäuse montiert wird und ohne Klemmbügel verrutschen kann. Das Halterungselement (14) ist zudem so ausgelegt, dass es Fertigungstoleranzen des Displays (13) durch eine unterschiedliche Spannung des Klemmbügels (14a) ausgleichen kann. Die Einzelteile sind in Fig.9 und die vormontierte Einheit aus Display (13) und Halterungselement (14) ist in Fig. 10 dargestellt. Die vormontierte Einheit wird unter Verwendung von Schrauben (15) gegenüber dem Gehäuse (5) verspannt. Vorzugsweise ist zwischen dem Display (13) und dem Gehäuse (1a) eine Dichtung (14b) angeordnet. Bei Verwendung eines anderen Displays (13) z.B. eines anderen Herstellers braucht in dieser Ausführungsform nur das Halterungselement (14) an das Display angepasst werden, die Gehäusekonstruktion kann beibehalten werden.
Beispielseweise kann das Display auch von außen in die Fronteinheit eingesetzt werden, wobei der Rahmen dann integraler Bestandteil dieses Vorderteils ist. In dieser Ausführungsform wird das Vorderteil unter der zusätzlichen Verwendung einer Frontfolie oder Bedienfolie ausgeführt, die ebenfalls von vorn auf das Frontelement aufgesetzt wird, wodurch das Display zwischen dem Gehäuseteil und der Frontfolie aufgenommen uns gehalten wird. In einer wiederum bevorzugten Ausführungsform überdeckt die Bedienfolie im Wesentlichen den von außen sichtbaren Teil des Vorderteils. Alternativ oder in Ergänzung zu den weiter oben beschriebenen berührempfindlichen Flächen auf dem Display können weitere Bedienfunktioen in die Frontfolie integriert sein.

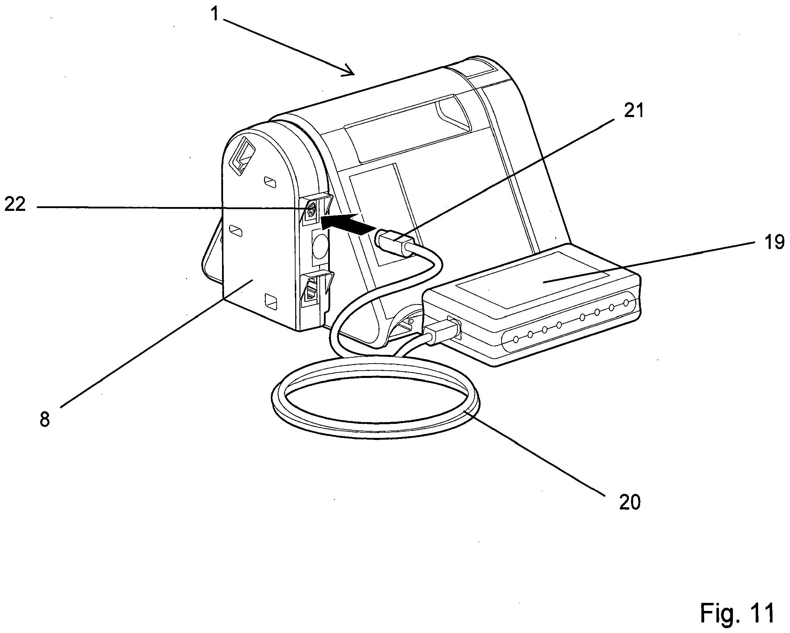

Fig. 11 zeigt eine Ausführungsform, bei der das Beatmungsgerät (1) mit einem als Kommunikationsmodul ausgebildeten Zusatzelement (8) versehen ist. Das Zusatzelement (8) dient hier zur Verbindung mit einem Polysomnographie-Gerät (PSG) (19). Eine Ankopplung des PSG (19) kann unter Verwendung eines Kabels (20) erfolgen, das über einen Stecker (21) mit einer Schnittstelle (22) des Zusatzelementes (8) koppelbar ist.

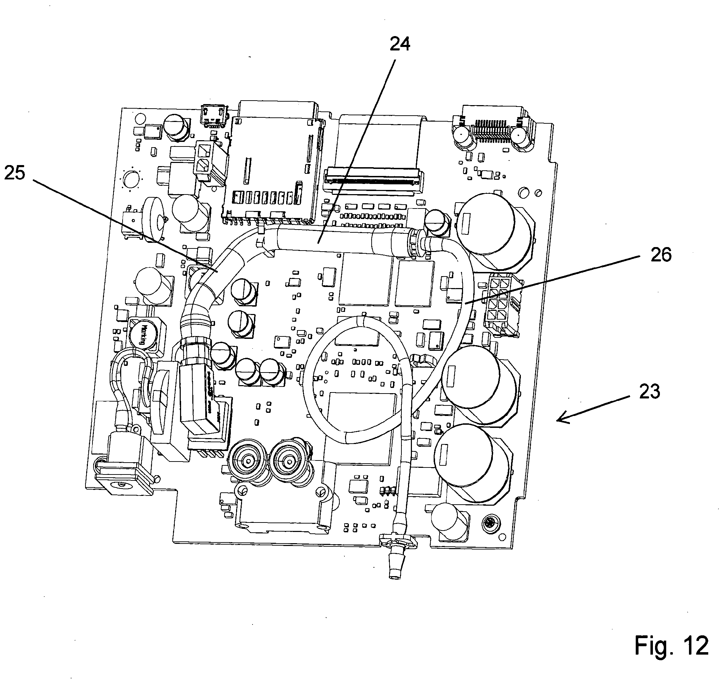

Fig. 12 zeigt eine Draufsicht auf eine Steuerungsplatine (23), die innerhalb der Fronteinheit (1a) des Beatmungsgerätes (1) anordbar ist. Die Steuerungsplatine (23) unterstützt eine Druckmessung und ist zur Weiterleitung des Druckes mit einem steckbaren Schlauch (24) versehen.

Gemäß dem dargestellten Ausführungsbeispiel besteht der Schlauch (24) aus einem ersten Schlauchabschnitt (25) mit einem ersten Durchmesser aus und einem zweiten Schlauchabschnitt (26) mit einem zweiten Durchmesser. Der erste Durchmesser ist größer als der zweite Durchmesser.

Durch die Kombination des ersten Schlauchabschnittes (25) und des zweiten Schlauchabschnittes (26) wird eine Signalfilterung bereitgestellt, die insbesondere im Drucksignal vorhandene Oberwellen herausfiltert. Alternativ zur mechanischen Realisierung des Filters über die Schlauchabschnitte (25, 26) ist grundsätzlich auch eine elektrische, elektronische oder Softwarebasierte Filterung möglich.

## Patentansprüche

1. Vorrichtung zur Beatmung, die ein mit einer Steuerung (23) verbundenes Gebläse (9) aufweist, wobei sowohl die Steuerung als auch das Gebläse in einem Gehäuse (1) angeordnet sind, und wobei die Steuerung mit mindestens einer Anzeigeeinrichtung (2) sowie mindestens einem Bedienelement (3) verbunden ist,
wobei das Gehäuse (1) aus einer Fronteinheit (1a) besteht, die mindestens eine Anzeigeeinrichtung (2) aufweist, und das Gehäuse (1) zwei weitere Gehäuseteile (16, 17) aufweist welche eine Gebläsebox (5) ausbilden,
wobei das Gebläse (9) im Gehäuse (1,16,17) von einem viskoelastischen oder elastomeren Tragteil (11) gehaltert ist,
wobei das Tragteil (11) in vertikaler Richtung zwischen den Gehäuseteilen (16, 17) angeordnet ist, **dadurch gekennzeichnet, dass** die Fronteinheit aus einem sich vertikal erstreckenden ersten Segment und einem zweiten zur vertikalen Richtung geneigten Segment besteht, in dem die mindestens eine Anzeigeeinrichtung (2) angeordnet ist, wobei die mindestens eine Anzeigeeinrichtung (2) geneigt zur vertikalen Richtung angeordnet ist.

2. Vorrichtung nach Anspruch 1, **dadurch gekennzeichnet, dass** das Tragteil (11) zwischen den Gehäuseteilen (16, 17) gehaltert ist, wobei die beiden Gehäuseteile (16, 17) jeweils die anteiligen Kammerhälften von Hochdruckbereich (16.1, 17.1) und Ansaugbereich (16.2, 17.2) aufweisen.

3. Vorrichtung nach zumindest einem der vorhergehenden Ansprüche, **dadurch gekennzeichnet, dass** das Tragteil (11) eine erste Dichtung (30) ausbildet die gegen Dichtflächen (31, 32) der Gehäuseteile (16 und 17) abdichtet und das Tragteil (11) eine zweite Dichtung (33) ausbildet, die gegen die Dichtflächen (34, 35) der beiden Gehäuseteile (16, 17) abdichtet und so den Hochdruckbereich (16.1, 17.1) vom Ansaugbereich (16.2, 17.2) pneumatisch trennt.

4. Vorrichtung nach zumindest einem der vorhergehenden Ansprüche, **dadurch gekennzeichnet, dass** das Tragteil (11) in der Gebläsebox (5) der Luftumlenkung und somit der Schallreduzierung dient und dazu zumindest zwei Öffnungen (11.1, 11.2, 11.3, 11.4) aufweist.

5. Vorrichtung nach zumindest einem der vorhergehenden Ansprüche, **dadurch gekennzeichnet, dass** das Tragteil (11) zumindest eine Öffnung (11.4) aufweist, die zumindest abschnittsweise von einem Kragen (28) umgeben ist.

6. Vorrichtung nach zumindest einem der vorhergehenden Ansprüche, **dadurch gekennzeichnet, dass** innerhalb der Gebläsebox (5) für eine Atemgasströmung mindestens drei Umlenkungen (A, B, C, D, E, F, G, H, I) angeordnet sind.

7. Vorrichtung nach zumindest einem der vorhergehenden Ansprüche, **dadurch gekennzeichnet, dass** innerhalb der Gebläsebox (5) und/oder am Tagteil (11) mindestens ein schalldämmendes Element (11', 11", 12, 17', 28) angeordnet ist.

8. Vorrichtung nach zumindest einem der vorherigen Ansprüche, **dadurch gekennzeichnet**, das in der Fronteinheit (1a) die Anzeigeeinrichtung (2) sowie eine Frontfolie aufgenommen ist, die über mindestens ein Bedienelement verfügt.

9. Vorrichtung nach zumindest einem der vorherigen Ansprüche, **dadurch gekennzeichnet, dass** das Gehäuse eine dreieckige Querschnittfläche aufweist

10. Vorrichtung nach zumindest einem der vorherigen Ansprüche, **dadurch gekennzeichnet**, das lediglich ein mechanisches Bedienelement (3) zur Aktivierung einer Gerätefunktion vorgesehen ist.

11. Vorrichtung nach zumindest einem der vorherigen Ansprüche, **dadurch gekennzeichnet**, das ergänzend zum lediglich einen mechanischen Bedienelement (3) weitere Bedienfelder auf dem als Touchscreen ausgebildeten Display (13) vorgesehen sind.

## Claims

1. A ventilation device that has a fan (9) connected to a control (23), wherein both the control as well as the fan are arranged in a housing (1), and wherein the control is connected to at least one display apparatus (2) as well as to the at least one control element (3), wherein the housing (1) consists of a front unit (1a) that has at least one display apparatus (2), and the housing (1) has two additional housing parts (16, 17) that form a fan box (5), wherein the fan (9) is held in the housing (1, 16, 17) by a viscoelastic or elastomer support part (11), wherein the support part (11) is arranged in a vertical direction between the housing parts (16, 17), **characterized in that** the front unit consists of a vertically extending first segment and a second segment that is oblique to the vertical direction and in which the at least one display apparatus (2) is arranged, wherein the at least one display apparatus (2) is arranged oblique to the vertical direction.

2. The device according to claim 1, **characterized in that** the support part (11) is held between the housing parts (16, 17), wherein the two housing parts (16, 17) each have the proportionate chamber halves of the high pressure region (16.1, 17.1) and suction region (16.2, 17.2) . '

3. The device according to at least one of the preceding claims, **characterized in that** the support part (11) forms a first seal (30) that seals against sealing surfaces (31, 32) of the housing parts (16 and 17), and the support part (11) forms a second seal (33) that seals against the sealing surfaces (34, 35) of the two housing parts (16, 17) and thereby pneumatically separates the high pressure region (16.1, 17.1) from the suction region (16.2, 17.2).

4. The device according to at least one of the preceding claims, **characterized in that** the support part (11) in the fan box (5) serves to deflect air and hence to reduce noise, and has at least two openings (11.1, 11.2, 11.3, 11.4) for this purpose.

5. The device according to at least one of the preceding claims, **characterized in that** the support part (11) has at least one opening (11.4) that is surrounded at least sectionally by a collar (28).

6. The device according to at least one of the preceding claims, **characterized in that** at least three deflections (A, B, C, D, E, F, G, H, I) are arranged within the fan box (5) for a respiratory gas flow.

7. The device according to at least one of the preceding claims, **characterized in that** at least one noise dampening element (11', 11", 12, 17', 28) is arranged within the fan box (5) and/or on the support part (11) .

8. The device according to at least one of the preceding claims, **characterized in that** in the front unit (1a) the display apparatus (2) as well as a front film are accommodated, which has at least one control element.

9. The device according to at least one of the preceding claims, **characterized in that** the housing has a triangular cross-sectional surface.

10. The device according to at least one of the preceding claims, **characterized in that** only one mechanical control element (3) is provided to activate a device function.

11. The device according to at least one of the preceding claims, **characterized in that** additional control fields are provided on the display (13) designed as a touchscreen in addition to only one mechanical control element (3).

## Revendications

1. Dispositif respiratoire présentant un ventilateur (9) relié à une commande (23), dans lequel aussi bien la commande que le ventilateur sont disposés dans un boîtier (1), et dans lequel la commande est reliée à au moins un dispositif d'affichage (2) et à au moins un élément de commande (3), dans lequel le boîtier (1) est constitué d'une unité frontale (1a) présentant au moins un dispositif d'affichage (2), et le boîtier (1) présente deux autres parties de boîtier (16, 17) formant une caisse de ventilateur (5), dans lequel le ventilateur (9) est maintenu dans le boîtier (1, 16, 17) par une pièce de support (11) viscoélastique ou élastomère, dans lequel la pièce de support (11) est disposée entre les parties de boîtier (16, 17) dans la direction verticale, **caractérisé en ce que** l'unité frontale est constituée d'un premier segment s'étendant verticalement et d'un deuxième segment incliné par rapport à la direction verticale, dans lequel l'au moins un dispositif d'affichage (2) est disposé, dans lequel l'au moins un dispositif d'affichage (2) est disposé de façon inclinée par rapport à la direction verticale.

2. Dispositif selon la revendication 1, **caractérisé en ce que** la pièce de support (11) est maintenue entre les parties de boîtier (16, 17), dans lequel les deux parties de boîtier (16, 17) présentent respectivement les moitiés de chambre correspondantes de la région de haute pression (16.1, 17.1) et de la région d'aspiration (16.2, 17.2).

3. Dispositif selon l'une au moins des revendications précédentes, **caractérisé en ce que** la pièce de support (11) forme une première garniture d'étanchéité (30) assurant l'étanchéité par rapport à des surfaces d'étanchéité (31, 32) des parties de boîtier (16 et 17) et **en ce que** la pièce de support (11) forme une deuxième garniture d'étanchéité (33) assurant l'étanchéité par rapport aux surfaces d'étanchéité (34, 35) des deux parties de boîtier (16, 17) et séparant de manière pneumatique ainsi la région de haute pression (16.1, 17.1) d'avec la région d'aspiration (16.2, 17.2) .

4. Dispositif selon l'une au moins des revendications précédentes, **caractérisé en ce que** la pièce de support (11) sert à dévier l'air dans la caisse de ventilateur (5) et donc à réduire le bruit, et présente au moins deux ouvertures (11.1, 11.2, 11.3, 11.4) prévues à cet effet.

5. Dispositif selon l'une au moins des revendications précédentes, **caractérisé en ce que** la pièce de support (11) présente au moins une ouverture (11.4), laquelle est entourée au moins par endroits d'un col (28).

6. Dispositif selon l'une au moins des revendications précédentes, **caractérisé en ce qu'**au moins trois déviations (A, B, C, D, E, F, G, H, I) sont disposées à l'intérieur de la caisse de ventilateur (5) pour un flux de gaz respiratoire.

7. Dispositif selon l'une au moins des revendications précédentes, **caractérisé en ce qu'**au moins un élément insonorisant (11', 11", 12, 17', 28) est disposé à l'intérieur de la caisse de ventilateur (5) et/ou sur la pièce de support (11).

8. Dispositif selon l'une au moins des revendications précédentes, **caractérisé en ce que** le dispositif d'affichage (2) ainsi qu'une feuille frontale disposant d'au moins un élément de commande sont reçus dans l'unité frontale (1a).

9. Dispositif selon l'une au moins des revendications précédentes, **caractérisé en ce que** le boîtier présente une section transversale triangulaire.

10. Dispositif selon l'une au moins des revendications précédentes, **caractérisé en ce qu'**il n'est prévu qu'un seul élément de commande mécanique (3) pour l'activation d'une fonction d'appareil.

11. Dispositif selon l'une au moins des revendications précédentes, **caractérisé en ce que** d'autres champs de commande sont prévus sur l'affichage (13) conçu comme un écran tactile, en complément du seul élément de , commande mécanique (3).
